# EUROPEAN PATENT APPLICATION

(11) **EP 4 292 661 A1**
(43) Date of publication of application: **20.12.2023**
(21) Application number: 22752269.5
(22) Date of filing: 09.02.2022
(51) Int. Cl.: A61P 35/00, C07K 16/22, A61K 39/395, C12P 21/08

(54) **ANTI-VEGF ANTIBODY AND USE THEREOF**

(30) Priority: 10.02.2021 CN 202110183560
(71) Applicant: Shanghai Jemincare Pharmaceuticals Co., Ltd., Pudong New Area, Shanghai 201203 (CN); Jiangxi Jemincare Group Co., Ltd., Nanchang, Jiangxi 330096 (CN)
(72) Inventor: WANG, Zongda, Shanghai 201203 (CN); GU, Chunyin, Shanghai 201203 (CN); CAO, Xiaodan, Shanghai 201203 (CN); LIU, Xiaowu, Shanghai 201203 (CN); DENG, Sujun, Shanghai 201203 (CN); PAN, Zhongzong, Shanghai 201203 (CN); WANG, Xueping, Shanghai 201203 (CN)
(74) Representative: Sagittarius IP
(86) International application number: PCT/CN2022/075600
(87) International publication number: WO 2022/171109

(57) **Abstract**

Provided is an isolated antigen binding protein capable of binding to VEGF protein and/or capable of blocking binding of VEGF protein to VEGFR protein. Further provided is a use of the isolated antigen binding protein in the preparation of a drug.

## Description

### FIELD OF THE INTVENTION

The present application relates to the field of biomedicine, in particular to an anti-VEGF antibody and use thereof in the preparation of medicaments.

### BACKGROUND OF THE INVENTION

Malignant tumor is currently a disease that seriously threatens human health worldwide, and is the main type of disease that causes human death. With the increasing aging of the domestic population and the increasing incidence of tumors, effective therapeutic drugs are urgently needed to be developed, among which the development of anti-angiogenic drugs has become a research hotspot in recent years. The growth and metastasis of tumors require abundant blood vessels to provide sufficient oxygen and nutrients, and tumor tissue can secrete a variety of pro-angiogenic substances.

During tumor growth, the epidermal growth factor VEGF can specifically stimulate the proliferation of endothelial cells, and plays a key role in various types of tumor angiogenesis. After binding to the epidermal growth factor receptor VEGFR, VEGF can mediate the transcription and expression of intracellular related protein genes through downstream signaling pathways, and promote the proliferation of vascular endothelial cells. At present, a number of humanized monoclonal antibodies targeting human VEGF, such as Bevacizumab, have been developed, but there are still such phenomena as low response rate and easy to produce drug resistance during the treatment process. Therefore, there is an urgent need for anti-tumor VEGF antibodies with stable structure, good efficacy and suitable for large-scale industrial production.

### SUMMARY OF THE INVENTION

The present application provides an isolated antigen-binding protein having one or more of the following properties: (1) capable of binding VEGF protein with a *K_{D}* value of 1×10⁻⁷ M or lower; and (2) capable of blocking the binding of VEGF protein to VEGFR protein.

In certain embodiments, the VEGF protein includes VEGF 165.

In certain embodiments, the VEGFR protein includes VEGFR2.

In certain embodiments, the isolated antigen-binding protein includes an antibody or an antigen-binding fragment thereof.

In certain embodiments, the antigen-binding fragment includes Fab, Fab', F(ab)₂, Fv fragment, F(ab')₂, scFv, di-scFv, VHH and/or dAb.

In certain embodiments, the antigen-binding fragment is VHH.

In certain embodiments, the antibody is selected from the group consisting of: a monoclonal antibody, a chimeric antibody, a humanized antibody and a fully human antibody.

In certain embodiments, the isolated antigen-binding protein is capable of competing with a reference antibody for binding to the VEGF protein, wherein the reference antibody comprises a heavy chain variable region VH, the VH of the reference antibody comprises HCDR1, HCDR2 and HCDR3, and the HCDR1 of the reference antibody comprises an amino acid sequence set forth in SEQ ID NO: 9, the HCDR2 of the reference antibody comprises an amino acid sequence set forth in SEQ ID NO: 23, and, the HCDR3 of the reference antibody comprises an amino acid sequence set forth in SEQ ID NO: 24.

In certain embodiments, the HCDR1 of the reference antibody comprises an amino acid sequence set forth in SEQ ID NO: 9.

In certain embodiments, the HCDR2 of the reference antibody comprises an amino acid sequence set forth in any one of SEQ ID NOs: 12-14.

In certain embodiments, the HCDR3 of the reference antibody comprises an amino acid sequence set forth in any one of SEQ ID NOs: 17-19.

In certain embodiments, the isolated antigen-binding protein comprises at least one CDR in a VH, the VH comprising an amino acid sequence set forth in SEQ ID NO: 29.

In certain embodiments, the isolated antigen-binding protein comprises at least one CDR in a VH, the VH comprising an amino acid sequence set forth in any one of SEQ ID NOs: 1-6.

In certain embodiments, the isolated antigen-binding protein comprises HCDR3, and the HCDR3 comprises an amino acid sequence set forth in SEQ ID NO: 24.

In certain embodiments, the HCDR3 comprises an amino acid sequence set forth in any one of SEQ ID NOs: 17-19.

In certain embodiments, the isolated antigen-binding protein comprises HCDR2, and the HCDR2 comprises an amino acid sequence set forth in SEQ ID NO: 23.

In certain embodiments, the HCDR2 comprises an amino acid sequence set forth in any one of SEQ ID NOs: 12-14.

In certain embodiments, the isolated antigen-binding protein comprises HCDR1, and the HCDR1 comprises an amino acid sequence set forth in SEQ ID NO: 9.

In certain embodiments, the isolated antigen-binding protein comprises HCDR1, HCDR2 and HCDR3, wherein the HCDR1 comprises an amino acid sequence set forth in SEQ ID NO: 9, the HCDR2 comprises an amino acid sequence set forth in SEQ ID NO: 23, and the HCDR3 comprises an amino acid sequence set forth in SEQ ID NO: 24.

In certain embodiments, the isolated antigen-binding protein comprises HCDR1, HCDR2 and HCDR3, wherein the HCDR1 comprises an amino acid sequence set forth in SEQ ID NO: 9, the HCDR2 comprises an amino acid sequence set forth in any one of SEQ ID NOs: 12-14, and the HCDR3 comprises an amino acid sequence set forth in any one of SEQ ID NOs: 17-19.

In certain embodiments, the isolated antigen-binding protein comprises HCDR1, HCDR2 and HCDR3, and the HCDR1, HCDR2 and HCDR3 are selected from any one of the following groups of amino acid sequences:
(1) HCDR1: SEQ ID NO: 9, HCDR2: SEQ ID NO: 12 and HCDR3: SEQ ID NO: 17;
(2) HCDR1: SEQ ID NO: 9, HCDR2: SEQ ID NO: 13 and HCDR3: SEQ ID NO: 17;
(3) HCDR1: SEQ ID NO: 9, HCDR2: SEQ ID NO: 14 and HCDR3: SEQ ID NO: 17;
(4) HCDR1: SEQ ID NO: 9, HCDR2: SEQ ID NO: 13 and HCDR3: SEQ ID NO: 18; and
(5) HCDR1: SEQ ID NO: 9, HCDR2: SEQ ID NO: 13 and HCDR3: SEQ ID NO: 19.

In certain embodiments, the isolated antigen-binding protein comprises a heavy chain variable region VH, wherein the VH comprises a framework region H-FR1, the C-terminus of the H-FR1 is directly or indirectly linked to the N-terminus of the HCDR1, and the H-FR1 comprises an amino acid sequence set forth in SEQ ID NO: 25.

In certain embodiments, the H-FR1 comprises an amino acid sequence set forth in any one of SEQ ID NOs: 7-8.

In certain embodiments, the VH comprises a framework region H-FR2, the H-FR2 is located between the HCDR1 and the HCDR2, and the H-FR2 comprises an amino acid sequence set forth in SEQ ID NO: 26.

In certain embodiments, the H-FR2 comprises an amino acid sequence set forth in any one of SEQ ID NOs: 10-11.

In certain embodiments, the VH comprises a framework region H-FR3, the H-FR3 is located between the HCDR2 and the HCDR3, and the H-FR3 comprises an amino acid sequence set forth in SEQ ID NO: 27.

In certain embodiments, the H-FR3 comprises an amino acid sequence set forth in any one of SEQ ID NO: 15-16.

In certain embodiments, the N-terminus of the H-FR4 is linked to the C-terminus of the HCDR3, and the H-FR4 comprises an amino acid sequence set forth in SEQ ID NO: 28.

In certain embodiments, the H-FR4 comprises an amino acid sequence set forth in any one of SEQ ID NOs: 20-22.

In certain embodiments, the isolated antigen-binding protein comprises H-FR1, H-FR2, H-FR3 and H-FR4, wherein the H-FR1 comprises an amino acid sequence set forth in SEQ ID NO: 25, the H-FR2 comprises an amino acid sequence set forth in SEQ ID NO: 26, the H-FR3 comprises an amino acid sequence set forth in SEQ ID NO: 27 and the H-FR4 comprises an amino acid sequence set forth in SEQ ID NO: 28.

In certain embodiments, the isolated antigen-binding protein comprises H-FR1, H-FR2, H-FR3 and H-FR4, wherein the H-FR1 comprises an amino acid sequence set forth in SEQ ID NO: 7 or SEQ ID NO: 8, the H-FR2 comprises an amino acid sequence set forth in SEQ ID NO: 10 or SEQ ID NO: 11, the H-FR3 comprises an amino acid sequence set forth in SEQ ID NO: 15 or SEQ ID NO: 16, and the H-FR4 comprises an amino acid sequence set forth in any one of SEQ ID NOs: 20-22.

In certain embodiments, the isolated antigen-binding protein comprises H-FR1, H-FR2, H-FR3 and H-FR4, and the H-FR1, H-FR2, H-FR3 and H-FR4 are selected from any one of the following groups of amino acid sequences:
(1) F-FR1: SEQ ID NO: 7, H-FR2: SEQ ID NO: 10, H-FR3: SEQ ID NO: 15 and H-FR4: SEQ ID NO: 20;
(2) H-FR1: SEQ ID NO: 8, H-FR2: SEQ ID NO: 11, H-FR3: SEQ ID NO: 16 and H-FR4: SEQ ID NO: 21; and
(3) H-FR1: SEQ ID NO: 8, H-FR2: SEQ ID NO: 11, H-FR3: SEQ ID NO: 16 and H-FR4: SEQ ID NO: 22.

In certain embodiments, the isolated antigen-binding protein comprises a heavy chain variable region VH, and the VH comprises an amino acid sequence set forth in SEQ ID NO: 29.

In certain embodiments, the VH comprises an amino acid sequence set forth in any one of SEQ ID NOs: 1-6.

In another aspect, the present application provides one or more isolated nucleic acid molecules encoding the isolated antigen-binding protein.

In another aspect, the present application provides a vector comprising the nucleic acid molecule.

In another aspect, the present application provides a cell comprising the nucleic acid molecule or the vector.

In another aspect, the present application provides a method for preparing the isolated antigen-binding protein, the method comprising culturing the cell under such a condition that the isolated antigen-binding protein is expressed.

In another aspect, the present application provides a pharmaceutical composition comprising the isolated antigen-binding protein, the nucleic acid molecule, the vector and/or the cell, and optionally a pharmaceutically acceptable carrier.

In another aspect, the present application provides a polypeptide comprising the isolated antigen-binding protein.

In another aspect, the present application provides an immunoconjugate comprising the isolated antigen-binding protein or the polypeptide.

In another aspect, the present application provides the use of the isolated antigen-binding protein, the nucleic acid molecule, the vector, the cell, the pharmaceutical composition, the polypeptide and/or the immunoconjugate in the preparation of a medicament for preventing, alleviating and/or treating tumors.

In certain embodiments, the tumor includes a VEGF-overexpressing tumor.

In certain embodiments, the tumor includes a solid tumor and/or a non-solid tumor.

In certain embodiments, the tumor includes lung cancer, colorectal cancer, breast cancer, kidney cancer, gastric cancer, liver cancer, blastoma, cervical cancer and/or ovarian cancer.

In another aspect, the present application provides a method for preventing, alleviating or treating tumors, the method comprising administering the isolated antigen-binding protein, the nucleic acid molecule, the vector, the cell, the pharmaceutical composition, the polypeptide and/or the immunoconjugate to a subject in need thereof.

In certain embodiments, the tumor includes a VEGF-overexpressing tumor.

In certain embodiments, the tumor includes a solid tumor and/or a non-solid tumor.

In certain embodiments, the tumor includes lung cancer, colorectal cancer, breast cancer, kidney cancer, gastric cancer, liver cancer, blastoma, cervical cancer and/or ovarian cancer.

In another aspect, the present application provides the isolated antigen-binding protein, the nucleic acid molecule, the vector, the cell, the pharmaceutical composition, the polypeptide and/or the immunoconjugate for use in preventing, alleviating or treating tumors.

In certain embodiments, the tumor includes a VEGF-overexpressing tumor.

In certain embodiments, the tumor includes a solid tumor and/or a non-solid tumor.

In certain embodiments, the tumor includes lung cancer, colorectal cancer, breast cancer, kidney cancer, gastric cancer, liver cancer, blastoma, cervical cancer and/or ovarian cancer.

In another aspect, the present application provides a method for inhibiting the binding of VEGF protein to VEGFR protein, comprising administering the isolated antigen-binding protein, the nucleic acid molecule, the vector, the cell, the pharmaceutical composition, the polypeptide and/or the immunoconjugate.

Other aspects and advantages of the present application may be readily perceived by those skilled in the art from the detailed description below. The detailed description below only illustrates and describes the exemplary embodiments of the present application. As would be appreciated by those skilled in the art, the content of the present application allows those skilled in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the present invention involved in the present application are listed in the appended claims. The characteristics and advantages of the present invention involved in the present application may be better understood by referring to the exemplary embodiments and the accompanying drawings described in detail below. A brief description of the drawings is as follows:
Figure 1 shows a schematic diagram of the yeast display library construction method.
Figure 2 shows that the antigen-binding proteins Ab1910VE8, Ab1910VE9 and Ab1910VE6 described in the present application block the binding of human VEGF 165 to human VEGFR2.
Figure 3 shows that the antigen-binding proteins Ab1910VE18, Ab1910VE21 and Ab1910VE24 described in the present application block the binding of human VEGF165 to human VEGFR2.

### DETAILED DESCRIPTION OF THE PREFERRED EMBODIMENTS

The embodiments of the invention of the present application will be illustrated by specific examples below. Those skilled in the art can readily understand other advantages and effects of the present application from the content disclosed in the specification.

### Terms & Definitions

In the present application, the term "VEGF" generally refers to vascular endothelial cell growth factor, including its naturally occurring allelic and processed forms. The VEGF may include VEGF-A, VEGF-B, VEGF-C, VEGF-D, VEGF-E, VEGF-F and/or PIGF. In the present application, the VEGF may be VEGF-A, which may also be referred to as VEGF165. VEGF-A can bind to receptor tyrosine kinases, namely VEGFR-1 (Flt-1), VEGFR-2 (Flk-1/KDR), VEGFR3 and Neuropilin-1 (NRP-1). After binding to VEGFR2, VEGF165 can mediate the transcription and expression of intracellular related protein genes through the downstream PLC-γ-PKC-Raf-MEK-MAPK signaling pathway, and promote the proliferation of vascular endothelial cells (Takahashi T et al., Oncogene, 18(13):2221- 2230. (1999)). The term "VEGF" may also refer to VEGF from non-human species such as mice, rats or primates. In the present application, VEGF from a specific species can be expressed as follows, hVEGF means human VEGF, and mVEGF means murine VEGF. Generally, VEGF may refer to human VEGF. The term "VEGF" is also used to indicate a truncated form or fragment of intact VEGF, and also includes functional variants, isoforms, species homologues, derivatives, analogs of VEGF, and analogs having at least one common epitope with VEGF. The sequences of VEGF are already known in the art. For example, exemplary full-length human VEGFA protein sequences can be found under NCBI accession numbers NP_001020537, NP_001020538, NP_001020539, NP_001020540 or NP_001020541.

In the present application, the term "VEGFR" generally refers to vascular endothelial growth factor receptor, including its naturally occurring allelic and processed forms. VEGFRs can include VEGFR1, CEGFR2, and VEGFR3, as well as other alternatively spliced variants. VEGFR can be membrane bound or soluble. The term "VEGFR" may also refer to VEGFR from non-human species such as mice, rats or primates. In the present application, VEGFR from a specific species can be expressed as follows, hVEGFR means human VEGFR, and mVEGFR means murine VEGFR. Typically, VEGFR may refer to human VEGFR. The term "VEGFR" is also used to indicate a truncated form or fragment of intact VEGFR, and also includes functional variants, isoforms, species homologues, derivatives, analogs of VEGFR, and analogs having at least one common epitope with VEGFR. The sequences of VEGFR are already known in the art. For example, an exemplary full-length human VEGFR2 protein sequence can be found under NCBI accession number NP_002244.

In the present application, the term "homologue" generally refers to an amino acid sequence or a nucleotide sequence having a certain homology with a wild-type amino acid sequence and a wild-type nucleotide sequence. The term "homology" may be equated with sequence "identity". Homologous sequences may include amino acid sequences that can be at least 80%, 85%, 90%, 99.1%, 99.2%, 99.3%, 99.4%, 99.5%, 99.6%, 99.7%, 99.8%, or 99.9% identical to the subject sequence. Generally, a homologue will comprise the same active site, etc., as the subject amino acid sequence. Homology can be considered in terms of similarity (i.e., amino acid residues with similar chemical properties/functions), or it can be expressed in terms of sequence identity. In the present application, reference to a sequence which has a percent identity to any one of the SEQ ID NOs of the amino acid sequence or nucleotide sequence refers to a sequence which has the stated percent identity over the entire length of the SEQ ID NO referred to.

In the present application, the term "antigen-binding protein" generally refers to a protein comprising a portion that binds to an antigen, and optionally a scaffold or skeleton portion that allows the portion that binds to the antigen to adopt a conformation that promotes the binding of the antigen-binding protein to the antigen. An antigen-binding protein may typically comprise an antibody light chain variable region (VL), an antibody heavy chain variable region (VH), or both of the above, and functional fragments thereof. The variable regions of the heavy and light chains contain binding domains that interact with an antigen. Examples of antigen-binding proteins include, but are not limited to, antibodies, antigen-binding fragments, immunoconjugates, multispecific antibodies (e.g., bispecific antibodies), antibody fragments, antibody derivatives, antibody analogs, or fusion proteins, etc., provided that they exhibit the desired antigen-binding activity.

In the present application, the term "antibody" generally refers to an immunoglobulin reactive with a specified protein or peptide or a fragment thereof. Antibodies can be those from any class, including but not limited to IgG, IgA, IgM, IgD, and IgE, and antibodies from any subclass (eg, IgG1, IgG2, IgG3, and IgG4). The antibody can have a heavy chain constant region selected from, for example, IgG1, IgG2, IgG3, or IgG4. The antibody may also have a light chain selected from, for example, kappa (κ) or lambda (λ). The antibodies of the present application can be derived from any species.

In the present application, the term "antigen-binding fragment" generally refers to a portion of an antibody molecule, the portion comprising amino acid residues that interact with an antigen and confer specificity and affinity for the antigen to the antibody. Examples of antigen-binding fragments may include, but are not limited to, Fab, Fab', F(ab)₂, Fv fragment, F(ab')₂, scFv, di-scFv and/or dAb. In the present application, the term "Fab" generally refers to a fragment comprising a heavy chain variable domain and a light chain variable domain, and also comprising a constant domain of light chain and the first constant domain of heavy chain (CH1); the term "Fab'" generally refers to a fragment that is different from Fab by the addition of a few residues (comprising one or more cysteines from the hinge region of an antibody) to the carboxyl terminus of the CH1 domain of the heavy chain; the term "F(ab')₂" generally refers to a dimer of Fab', comprising an antibody fragment in which two Fab fragments are linked by a disulfide bridge on the hinge region. The term "Fv" generally refers to the smallest antibody fragment that comprises a complete antigen recognition and binding site. In some cases, this fragment may consist of a dimer in which one heavy chain variable region and one light chain variable region are tightly non-covalently bound; and the term "dsFv" generally refers to a disulfide bond-stabilized Fv fragment, in which the bond between a single light chain variable region and a single heavy chain variable region is a disulfide bond. The term "dAb fragment" generally refers to an antibody fragment consisting of a VH domain. In the present application, the term "scFv" generally refers to a monovalent molecule formed by covalently linking and pairing one heavy chain variable domain with one light chain variable domain of an antibody by means of a flexible peptide linker; such scFv molecules may have a general structure: NH₂-VL-linker-VH-COOH or NH₂-VH-linker-VL-COOH. In the present application, the term "VHH" generally refers to an antibody comprising the variable antigen-binding domain of a heavy chain antibody (see Vanlandschoot P. et al., 2011, Antiviral Research 92, 389-407). VHH may also be referred to as Nanobody (Nb).

In the present application, the term "variable region" or "variable domain" generally refers to a heavy chain or light chain domain of an antibody that participates in the binding of the antibody to an antigen. In the present application, the term "variable" generally means that certain portions of a variable domain sequence of an antibody change strongly, which contributes to the binding and specificity of various specific antibodies to specific antigens thereof. The variability is not evenly distributed throughout the variable region of the antibody. It is concentrated in three segments in the light and heavy chain variable regions, called complementarity determining regions (CDRs) or hypervariable regions (HVRs), respectively referred to as LCDR1, LCDR2, LCDR3, HCDR1, HCDR2 and HCDR3. The more highly conserved portions in the variable domain are called the framework regions (FRs). Each of the variable domains of natural heavy chain and light chain comprises four FR regions (H-FR1, H-FR2, H-FR3, H-FR4, L-FR1, L-FR2, L-FR3, L-FR4), most of which adopt a β-sheet configuration and are connected by three CDR structure loop regions. The CDRs in each chain are in close proximity together by the FR regions, and together with the CDRs from the other chain form the antigen binding site of the antibody.

In the art, the variable regions of an antibody can be encoded or the CDRs of an antibody can be divided by a variety of methods, such as the Kabat numbering scheme and definition rules based on sequence variability (see, Kabat et al., Protein Sequences in Immunology, Fifth Edition, National Institutes of Health, Bethesda, Maryland (1991), Chothia numbering scheme and definition rules based on the location of structural loop regions (see, A1-Lazikani et al., JMol Biol 273:927-48, 1997), efranc et al's IMGT numbering scheme and definition rules based on amino acid sequence alignment of germline V genes, as well as Honneger's numbering scheme (AHo's), Martin numbering scheme, Gelfand numbering scheme, etc., see Mathieu Dondelinger et al., Understanding the Significance and Implications of Antibody Numbering and Antigen-binding Surface/Residue Definition, Front. Immunol., 16 October 2018.

In the present application, the term "monoclonal antibody" generally refers to an antibody obtained from a population of substantially homogeneous antibodies, i.e., the individual antibodies constituting the population are identical except for possible naturally occurring mutations and/or post-translational modifications (e.g., isomerization, amidation) that may be present in extremely small amounts. Monoclonal antibodies are highly specific for a single antigen site.

In the present application, the term "chimeric antibody" generally refers to an antibody in which the variable region is derived from one species and the constant region is derived from another species. Generally, the variable region is derived from an antibody ("parent antibody") of an experimental animal such as a rodent, and the constant region is derived from a human antibody, such that the possibility of causing an adverse immune response in an individual human by the resulting chimeric antibody is reduced as compared with the parental (for example, mouse-derived) antibody.

In the present application, the term "humanized antibody" generally refers to an antibody in which some or all of the amino acids outside the CDR of a non-human antibody (such as a mouse antibody) have been replaced by corresponding amino acids derived from human immunoglobulins. In the CDR, additions, deletions, insertions, substitutions, or modifications to the amino acids may also be allowed, as long as they still retain the capability of the antibody to bind to a specific antigen. The humanized antibody may optionally comprise at least a portion of a constant region of a human immunoglobulin. The "humanized antibody" reserves the antigen specificity similar to that of the original antibody. The "humanized" form of a non-human (for example, mouse antibody) antibody may minimally comprise a chimeric antibody derived from a non-human immunoglobulin sequence. In some cases, residues of CDR regions in a human immunoglobulin (receptor antibody) may be replaced with residues of CDR regions from a non-human species (donor antibody) (such as a mouse, a rat, a rabbit, or a non-human primate) with the desired properties, affinity, and/or capability. In some cases, residues of FR regions of the human immunoglobulin may be replaced with corresponding non-human residues. In addition, the humanized antibody may comprise an amino acid modification that is not present in the receptor antibody or in the donor antibody.

In the present application, the term "fully human antibody" generally refers to an antibody that is expressed by an animal by transferring a human antibody-encoding gene into a genetically engineered animal with an antibody gene-deficiency. All portions of the antibody (comprising the variable and constant regions of the antibody) are encoded by genes originating from humans. Methods for obtaining the fully human antibody in the art may include the phage display technology, the transgenic mouse technology, the ribosome display technology, the RNA-polypeptide technology, etc.

In the present application, the term "binding", "specific binding", or "specific to ..." generally refers to a measurable and reproducible interaction, such as the binding between an antigen and an antibody, whereby the existence of a target may be determined in the presence of a heterogeneous population of molecules (including biomolecules). For example, an antibody binds to an epitope by means of its antigen-binding domain, and this binding requires some complementarity between the antigen-binding domain and the epitope. For example, an antibody that specifically binds to a target (which may be an epitope) is an antibody that binds to the target with greater affinity, avidity, easiness, and/or duration than it binds to other targets. When an antibody binds to an epitope by means of its antige-binding domain more easily than its binding to a random, unrelated epitope, the antibody is referred to as "specifically binding" to that antigen.

In the present application, the terms "KD", "*K_{D}*" used interchangeably and generally refer to the equilibrium dissociation constant. "KD" is the ratio of a dissociation rate constant (kdis, also known as "off-rate (koff)" or "kd") to an association rate constant (kon, also known as "on-rate (kon)" or "ka"). The association rate constant (kon), the dissociation rate constant (kdis), and the equilibrium dissociation constant (KD) can used to represent the binding affinity of an antigen-binding protein (for example, an antibody) to an antigen. Methods for determining the association and dissociation rate constants are well known in the art, including but not limited to the biofilm interference technology (BLI), the radioimmunoassay (RIA), the equilibrium dialysis, the surface plasmon resonance (SPR), the fluorescence resonance energy transfer (FRET), the co-immunoprecipitation (Co-IP), and the protein chip technology. If the measurement is performed under different conditions (for example, in terms of salt concentration and pH), the measured affinity of a specific protein-protein interaction may be different.

In the present application, the term "primate" usually refers to monkey and ape species, and includes monkey species, such as the monkeys from the Macaca (such as, cynomolgus (Macaca fascicularis ) and/or rhesus monkey (Macaca mulatta)) and baboon (Papio ursinus), as well as marmoset (a species from Callithrix), squirrel monkey (a species from Saimiri), and tamarin (a species from Saguinus), and ape species, such as chimpanzee (Pan troglodytes), and also includes Homo sapiens.

In the present application, the terms "polypeptide" or "protein" are used interchangeably to refer to a polymer of amino acid residues. The terms also apply to amino acid polymers in which one or more amino acid residues is an analog or mimetic of a corresponding naturally occurring amino acid, as well as to naturally occurring amino acid polymers. The terms can also encompass amino acid polymers that have been modified, e.g., by the addition of carbohydrate residues to form glycoproteins, or phosphorylated. Polypeptides and proteins can be produced by a naturally-occurring and non-recombinant cell or by a genetically-engineered or recombinant cell, and can comprise molecules having the amino acid sequence of the native protein, or molecules having deletions from, additions to, and/or substitutions of one or more amino acids of the native sequence. The terms "polypeptide" and "protein" specifically include, in particular, sequences that have deletions from, additions to, and/or substitutions of one or more amino acids of the antigen-binding proteins as described in the present application.

In the present application, the term "isolated" generally refers to a biological material, such as a virus, a nucleic acid or a protein, which is substantially free from components that normally accompany or interact with it in its naturally occurring environment. The isolated biological material optionally comprises additional material not found with the biological material in its natural environment, e.g., a nucleic acid or protein. In the present application, when a protein is referred to, "isolated" generally means that the molecule is isolated and separated from the entire organism in which the molecule is found to be naturally occurring, or means the substantial absence of other biological macromolecules of the same type. When a nucleic acid molecule is referred to, it is completely or partially separated from the sequence with which it is naturally associated, or the nucleic acid has a heterologous sequence associated with it, or the nucleic acid is isolated from the chromosome.

In the present application, the term "immunoconjugate" generally refers to a substance formed by linking an antigen-binding protein to other active agents, which may be small molecule active agents, such as chemotherapeutic agents, toxins, immunotherapeutic agents, imaging probes or spectroscopic probes.

In the present application, the term "nucleic acid" molecule generally refers to to any length of isolated form of a nucleotide, a deoxyribonucleotide or a ribonucleotide or an analog thereof that is isolated from the natural environment thereof or is artificially synthesized.

In the present application, the term "vector" generally refers to a nucleic acid molecule capable of self-replication in a suitable host, which transfers an inserted nucleic acid molecule into a host cell and/or among host cells. The vector may include a vector mainly for inserting DNA or RNA into cells, a vector mainly for replicating DNA or RNA, and a expression vector mainly for DNA or RNA transcription and/or translation. The vectors also include those having multiple functions of the above. The vectors may be polynucleotides that are capable of transcription and translation into polypeptides when introduced into suitable host cells. Generally, the vectors can produce desired expression products by culturing the suitable host cells comprising the vectors.

In the present application, the term "cell" generally refers to an individual cell, a cell line or a cell culture, which may contain or already contain a plasmid or vector comprising a nucleic acid molecule of the present application, or which is capable of expressing the antigen-binding protein in the present application. The cell may include a progeny of a single host cell. Due to natural, accidental or deliberate mutations, progeny cells and original parent cells may not be necessarily identical in terms of morphology or genome, as long as they are capable of expressing the antibody or antigen-binding fragment thereof in the present application. The cells may be obtained by transfecting cells *in vitro* using the vector of the present application. The cells may be prokaryotic cells (for example, *Escherichia coli*)*,* or eukaryotic cells (for example, yeast cells, for example, COS cells, Chinese hamster ovary (CHO) cells, HeLa cells, HEK293 cells, COS-1 cells, NSO cells, or myeloma cells). In some cases, the cells may be mammalian cells. For example, the mammalian cells may be CHO-K1 cells.

In the present application, the term "pharmaceutical composition" generally refers to a formulation, which exists in a form that allows the biological activity of the active ingredients to be effective, and does not comprise the additional ingredients that are unacceptably toxic to the subject to which the composition is administered.

In the present application, the term "treatment" generally refers to the desire to alter the natural course of a disease in an individual being treated, and may be a clinical intervention to achieve prevention and treatment or during the course of a clinical disease. Desirable therapeutic effects include, but are not limited to, preventing the occurrence or recurrence of disease, reducing symptoms, attenuating any direct or indirect pathological consequences of the disease, preventing metastasis, reducing the rate of disease progression, improving or ameliorating the disease state, and alleviating or improving prognosis. In some cases, antigen binding proteins (e.g., anti-VEGF antibodies) can be used to delay disease progression or slow disease progression.

In the present application, the term "administration" generally refers to a method of giving a certain dose of compound (e.g., an anti-tumor therapeutic agent) or pharmaceutical composition (e.g., a pharmaceutical composition comprising an anti-tumor therapeutic agent) to a subject (e.g., a patient). The administration can be carried out through any suitable means, including parenteral, intrapulmonary and intranasal, and (if topical treatment is needed) intralesional administration. Parenteral infusions include, for example, intramuscular, intravenous, intraarterial, intraperitoneal or subcutaneous administration.

In the present application, the term "tumor" generally refers to all neoplastic cell growth and proliferation (whether malignant or benign), and all precancerous and cancerous cells and tissues. In the present application, the tumor may be a tumor with high expression of VEGF or VEGFR in cells and tissues. Tumors can include solid tumors and/or non-solid tumors (e.g., hematological tumors, lymphomas).

In the present application, the term "between" generally means that the C-terminus of an amino acid fragment is directly or indirectly linked to the N-terminus of a first amino acid fragment, and the N-terminus thereof is directly or indirectly linked to the C-terminus of a second amino acid fragment. In a light chain, for example, the N-terminus of the L-FR2 is directly or indirectly linked to the C-terminus of the LCDR1, and the C-terminus of the L-FR2 is directly or indirectly linked to the N-terminus of the LCDR2. For another example, the N-terminus of the L-FR3 is directly or indirectly linked to the C-terminus of the LCDR2, and the C-terminus of the L-FR3 is directly or indirectly linked to the N-terminus of the LCDR3. In a heavy chain, for example, the N-terminus of the H-FR2 is directly or indirectly linked to the C-terminus of the HCDR1, and the C-terminus of the H-FR2 is directly or indirectly linked to the N-terminus of the HCDR2. For another example, the N-terminus of the H-FR3 is directly or indirectly linked to the C-terminus of the HCDR2, and the C-terminus of the H-FR3 is directly or indirectly linked to the N-terminus of the HCDR3. In the present application, the "first amino acid fragment" and the "second amino acid fragment" may be any amino acid fragments that are the same or different.

In the present application, the term "includes/include" generally refers to the meaning of comprising, inclusive, containing, or encompassing. In some cases, it also represents the meanings of "is", and "consist of".

In the present application, the term "about" generally refers to a range of 0.5%-10% of variation above or below a specific value, for example, a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of variation above or below a specific value.

### DETAILED DESCRIPTION OF THE INVENTION

### Antigen-binding protein

In one aspect, the present application provides an isolated antigen-binding protein capable of binding to a VEGF (e.g., VEGF165) derived from primates (e.g., humans) with a KD value of 1×10⁻⁸M or lower. The binding affinity of the VEGF antigen-binding protein for VEGF may be determined by any methods known in the art. In some cases, the binding affinity may be determined by Surface Plasmon Resonance (SPR), Enzyme-linked Immunosorbent Assay (ELISA), Binding Antigen Precipitation, Equilibrium Dialysis, Biolayer Interferometry (BLI). In some cases, the binding affinity and KD value of the VEGF antigen-binding protein for VEGF may be determined by Biolayer Interferometry (BLI). For example, ForteBio Octet molecular interaction analysis meter may be used to perform the analysis of binding dynamics between antigens and antibodies.

In the present application, the isolated antigen-binding protein can bind to a VEGF (e.g., VEGF165) with a KD value of 1×10⁻⁷ M or lower. For example, a VEGF derived from human may be bound with a KD value of about 5×10⁻⁸ M or less, about 4×10⁻⁸ M or less, about 3×10⁻⁸ M or less, about 2×10⁻⁸ M or less, about 1×10⁻⁸ M or less, about 9×10⁻⁹ M or less, about 8×10⁻⁹ M or less, about 7×10⁻⁹ M or less, about 6×10⁻⁹ M or less, about 5×10⁻⁹ M or less, about 4×10⁻⁹ M or less, about 3×10⁻⁹ M or less, about 2×10⁻⁹ M or less, about 1×10⁻⁹ M or less, about 9×10⁻¹⁰ M or less, about 8×10⁻¹⁰ M or less, about 7×10⁻¹⁰ M or less, e.g., as detected by ForteBio Octet molecular interaction analysis meter.

The antigen-binding proteins of the present application are capable of blocking the binding of VEGF (e.g., VEGF165) to VEGFR (e.g., VEGFR2). In some cases, the blocking of the binding of VEGF to VEGFR by the antigen-binding proteins may be determined by Flow Cytometry FACS, Enzyme-linked Immunosorbent assay ELISA
For example, human VEGFR (e.g., VEGFR2) is first incubated with a decreasing amount of the unlabelled antigen-binding protein, followed by incubation with labeled VEGF protein. Then, OD450 is measured to confirm that the antigen-binding protein blocks the binding of VEGF (e.g., VEGF165) to VEGFR (e.g., VEGFR2). For example, the IC50 for blocking activity is between about 0.001 µg/mL and about 10 µg/mL, between about 0.001 µg/mL and about 5 µg/mL, between about 0.01 µg/mL and about 1 µg/mL, between about 0.02 µg /mL and about 0.5 µg/mL, between about 0.2 µg/mL and about 15 µg/mL, between about 0.2 µg/mL and about 12 µg/mL, between about 0.2 µg/mL and about 10 µg/mL, between about 0.3 µg/mL and about 8 µg/mL, between about 0.3 µg/mL and about 6 µg/mL, between about 0.5 µg/mL and about 5 µg/mL, between about 0.1 µg/mL and about 2 µg/mL time, or between about 0.5 µg/mL and about 1.5 µg/mL.

In the present application, the isolated antigen-binding protein is capable of competing with a reference antibody for binding to the VEGF protein, wherein the reference antibody may comprise a heavy chain variable region VH, the VH of the reference antibody may comprise HCDR1, HCDR2 and HCDR3, and the HCDR1 of the reference antibody may comprise an amino acid sequence set forth in SEQ ID NO: 9, and the HCDR2 of the reference antibody may comprise an amino acid sequence set forth in SEQ ID NO: 23, and, the HCDR3 of the reference antibody may comprise an amino acid sequence set forth in SEQ ID NO: 24.

In the present application, the isolated antigen-binding protein is capable of competing with a reference antibody for binding to the VEGF protein, the reference antibody may comprise HCDR1, HCDR2 and HCDR3, and the HCDR1 of the reference antibody may comprise an amino acid sequence set forth in SEQ ID NO: 9, and the HCDR2 of the reference antibody may comprise an amino acid sequence set forth in any one of SEQ ID NOs: 12-14, and the HCDR3 of the reference antibody may comprise an amino acid sequence set forth in any one of SEQ ID NOs: 17-19.

In the present application, the isolated antigen-binding protein is capable of competing with a reference antibody for binding to the VEGF protein, the reference antibody may comprise HCDR1, HCDR2 and HCDR3, and the HCDR1, HCDR2 and HCDR3 of the reference antibody may be selected from any one of the following groups of amino acid sequences:
(1) HCDR1: SEQ ID NO: 9, HCDR2: SEQ ID NO: 12 and HCDR3: SEQ ID NO: 17;
(2) HCDR1: SEQ ID NO: 9, HCDR2: SEQ ID NO: 13 and HCDR3: SEQ ID NO: 17;
(3) HCDR1: SEQ ID NO: 9, HCDR2: SEQ ID NO: 14 and HCDR3: SEQ ID NO: 17;
(4) HCDR1: SEQ ID NO: 9, HCDR2: SEQ ID NO: 13 and HCDR3: SEQ ID NO: 18;
   and
(5) HCDR1: SEQ ID NO: 9, HCDR2: SEQ ID NO: 13 and HCDR3: SEQ ID NO: 19.

In the present application, the isolated antigen-binding protein may comprise at least one CDR in a VH, wherein the VH may comprise an amino acid sequence set forth in SEQ ID NO: 29.

In the present application, the isolated antigen-binding protein may comprise at least one CDR in a VH, wherein the VH may comprise an amino acid sequence set forth in any one of SEQ ID NOs: 1-6.

In the present application, the isolated antigen binding protein may be VHH.

In the present application, the isolated antigen binding protein may comprise HCDR3, which may comprise CDR3 of the VH whose amino acid sequence is set forth in SEQ ID NO: 29. In the present application, the isolated antigen-binding protein may comprise HCDR3, including CDR3 of the VH whose amino acid sequence is set forth in any one of SEQ ID NOs: 1-6.

For example, the isolated antigen-binding protein may comprise HCDR3, which may comprise an amino acid sequence set forth in SEQ ID NO: 24: RLRIX₅X₆X₇X₈X₉X₁₀ERLDY (SEQ ID NO: 24), wherein X₅ is P or T, X₆ is D or H, and X₇ is E, Q or W, X₈ is R, W or Y, X₉ is R or S, and X₁₀ is R or T. For example, the sequence may be a sequence determined according to the rules defined by Chothia.

In some cases, compared with the amino acid sequence set forth in SEQ ID NO: 17, the HCDR3 may at least comprise an amino acid substitution at a position selected from the following: amino acid substitutions at X₅, X₆, X₇, X₈, X₉ and/or X₁₀.

For example, the HCDR3 may comprise an amino acid sequence set forth in any one of SEQ ID NOs: 17-19.

In the present application, the isolated antigen-binding protein may comprise HCDR2, which may comprise CDR2 of the VH whose amino acid sequence is set forth in SEQ ID NO: 29. In the present application, the isolated antigen-binding protein may comprise HCDR2, which may comprise CDR2 of the VH whose amino acid sequence is set forth in any one of SEQ ID NOs: 1-6.

For example, the isolated antigen-binding protein may comprise HCDR2, which may comprise an amino acid sequence set forth in SEQ ID NO: 23: AVX₃AX₅X₆WX₈YVEDSVX₁₅G (SEQ ID NO: 23), wherein X₃ is F or L , X₅ is E or P, X₆ is D or G , X₈ is R or S, and X₁₅ is K or R. For example, the sequence may be a sequence determined according to the rules defined by Chothia.

In some cases, compared with the amino acid sequence set forth in SEQ ID NO: 12, the HCDR2 may at least comprise an amino acid substitution at a position selected from the following: amino acid substitutions at X₃, X₅, X₆, X₈ and/or X₁₅.

For example, the HCDR2 may comprise an amino acid sequence set forth in any one of SEQ ID NOs: 12-14.

In the present application, the isolated antigen-binding protein may comprise HCDR1, which may comprise CDR1 of the VH whose amino acid sequence is set forth in SEQ ID NO: 29. In the present application, the isolated antigen-binding protein may comprise HCDR1, which may comprise CDR1 of the VH whose amino acid sequence is set forth in any one of SEQ ID NOs: 1-6.

For example, the isolated antigen-binding protein may comprise HCDR1, which may comprise an amino acid sequence set forth in SEQ ID NO: 9.

In the present application, the isolated antigen-binding protein may comprise HCDR1, HCDR2 and HCDR3, the HCDR1 may comprise the CDR1 of the VH whose amino acid sequence is set forth in SEQ ID NO: 29, the HCDR2 may comprise the CDR2 of the VH whose amino acid sequence is set forth in SEQ ID NO: 29, and the HCDR3 may comprise the CDR3 of the VH whose amino acid sequence is set forth in SEQ ID NO: 29.

In the present application, the isolated antigen-binding protein may comprise HCDR1, HCDR2 and HCDR3, the HCDR1 may comprise the CDR1 of the VH whose amino acid sequence is set forth in any one of SEQ ID NOs: 1-6, the HCDR2 may comprise the CDR2 of the VH whose amino acid sequence is set forth in any one of SEQ ID NOs: 1-6, and the HCDR3 may comprise the CDR3 of the VH whose amino acid sequence is set forth in any one of SEQ ID NOs: 1-6.

In the present application, the isolated antigen-binding protein described in the present application may comprise HCDR1, HCDR2 and HCDR3, the HCDR1 may comprise an amino acid sequence set forth in SEQ ID NO: 9, the HCDR2 may comprise an amino acid sequence set forth in SEQ ID NO: 23, and the HCDR3 may comprise an amino acid sequence set forth in SEQ ID NO: 24.

In the present application, the isolated antigen-binding protein described in the present application may comprise HCDR1, HCDR2 and HCDR3, the HCDR1 may comprise an amino acid sequence set forth in SEQ ID NO: 9, the HCDR2 may comprise an amino acid sequence set forth in any one of SEQ ID NOs: 12-14, and the HCDR3 may comprise an amino acid sequence set forth in any one of SEQ ID NOs: 17-19.

For example, the isolated antigen-binding protein described in the application may comprise HCDR1, HCDR2 and HCDR3, the HCDR1 may comprise an amino acid sequence set forth in SEQ ID NO: 9, the HCDR2 may comprise an amino acid sequence set forth in SEQ ID NO: 12, and the HCDR3 may comprise an amino acid sequence set forth in SEQ ID NO: 17.

For example, the isolated antigen-binding protein described in the application may comprise HCDR1, HCDR2 and HCDR3, the HCDR1 may comprise an amino acid sequence set forth in SEQ ID NO: 9, the HCDR2 may comprise an amino acid sequence set forth in SEQ ID NO: 13, and the HCDR3 may comprise an amino acid sequence set forth in SEQ ID NO: 17.

For example, the isolated antigen-binding protein described in the application may comprise HCDR1, HCDR2 and HCDR3, the HCDR1 may comprise an amino acid sequence set forth in SEQ ID NO: 9, the HCDR2 may comprise an amino acid sequence set forth in SEQ ID NO: 14, and the HCDR3 may comprise an amino acid sequence set forth in SEQ ID NO: 17.

For example, the isolated antigen-binding protein described in the application may comprise HCDR1, HCDR2 and HCDR3, the HCDR1 may comprise an amino acid sequence set forth in SEQ ID NO: 9, the HCDR2 may comprise an amino acid sequence set forth in SEQ ID NO: 13, and the HCDR3 may comprise an amino acid sequence set forth in SEQ ID NO: 18.

For example, the isolated antigen-binding protein described in the application may comprise HCDR1, HCDR2 and HCDR3, the HCDR1 may comprise an amino acid sequence set forth in SEQ ID NO: 9, the HCDR2 may comprise an amino acid sequence set forth in SEQ ID NO: 13, and the HCDR3 may comprise an amino acid sequence set forth in SEQ ID NO: 19.

In the present application, the isolated antigen-binding protein may comprise H-FR1, which may comprise an amino acid sequence set forth in SEQ ID NO: 25: X₁VQLVESGGGLVQX₁₄GGSX₁₈RLSCAASGSTSD (SEQ ID NO: 25), wherein X₁ is A or E, X₁₄ is A or P, and X₁₈ is A or L. For example, the sequence may be a sequence determined according to the rules defined by Chothia.

In some cases, compared with the amino acid sequence set forth in SEQ ID NO: 7, the H-FR1 may at least comprise an amino acid substitution at a position selected from the following: amino acid substitutions at X₁, X₁₄ and/or X₁₈.

For example, the H-FR1 may comprise an amino acid sequence set forth in SEQ ID NO: 7 or 8. In the present application, the isolated antigen-binding protein may comprise H-FR2, which may comprise an amino acid sequence set forth in SEQ ID NO: 26: WYRQAPGKERXQQLVX₁₄ (SEQ ID NO: 26), wherein X₁₁ is D or E, and X₁₄ is A or S. For example, the sequence may be a sequence determined according to the rules defined by Chothia.

In some cases, compared with the amino acid sequence set forth in SEQ ID NO: 10, the H-FR2 may at least comprise an amino acid substitution at a position selected from the following: amino acid substitutions at X₁₁ and/or X₁₄.

For example, the H-FR2 may comprise an amino acid sequence set forth in SEQ ID NO: 10 or 11.

In the present application, the isolated antigen-binding protein may comprise H-FR3, which may comprise an amino acid sequence set forth in SEQ ID NO: 27:
RFTISRDNX₉KNTVX₁₄LQMNX₁₉LX₂₁X₂₂EDTAX₂₇YYCNV (SEQ ID NO: 27), wherein X₉ is S or T, X₁₄ is D or Y, X₁₉ is N or S, X₂₁ is K or R, X₂₂ is A or P, and X₂₇ is I or V For example, the sequence may be a sequence determined according to the rules defined by Chothia.

In some cases, compared with the amino acid sequence set forth in SEQ ID NO: 15, the H-FR3 may at least comprise an amino acid substitution at a position selected from the following: amino acid substitutions at X₉, X₁₄, X₁₉, X₂₁, X₂₂ and/or X₂₇.

For example, the H-FR3 may comprise an amino acid sequence set forth in SEQ ID NO: 15 or 16.

In the present application, the isolated antigen-binding protein may comprise H-FR4, which may comprise an amino acid sequence set forth in SEQ ID NO: 28: WGX3GTX6VTVSS (SEQ ID NO: 28), wherein X₃ is K or Q, and X₆ is L, Q or T. For example, the sequence may be a sequence determined according to the rules defined by Chothia.

In some cases, compared with the amino acid sequence set forth in SEQ ID NO: 20, the H-FR4 may at least comprise an amino acid substitution at a position selected from the following: amino acid substitutions at X₃ and/or X₆.

For example, the H-FR4 may comprise an amino acid sequence set forth in any one of SEQ ID NOs: 20-22.

In the present application, the isolated antigen-binding protein may comprise a heavy chain variable region VH, which may comprise an amino acid sequence set forth in SEQ ID NO: 29:
X₁VQLVESGGGLVQX₁₄GGSX₁₈RLSCAASGSTSDIVSMAWYRQAPGKERX₄₆LVX₄₉AVX_{5 2}AX₅₄X₅₅WX₅₇YVEDSVX₆₄GRFTISRDNX₇₄KNTVX₇₉LQMNX₈₄LX₈₆X₈₇EDTAX₉₂YYCNVRLR IX₁₀₂X₁₀₃X₁₀₄X₁₀₅X₁₀₆X₁₀₇ERLDYWGX₁₁₅GTX₁₁₈VTVSS (SEQ ID NO: 29), wherein X₁ is A or E, X₁₄ is A or P, X₁₈ is A or L, X₄₆ is D or E, X₄₉ is A or S, X₅₂ is F or L, X ₅₄ is E or P, X₅₅ is D or G, X₅₇ is R or S, X₆₄ is K or R, X₇₄ is S or T, X₇₉ is the D or Y, X₈₄ is N or S, X₈₆ is K or R, X₈₇ is A or P, X₉₂ is I or V, X₁₀₂ is P or T, X₁₀₃ is D or H, X₁₀₄ is E, Q or W, X₁₀₅ is the R, W or Y, X₁₀₆ are R or S, X₁₀₇ is R or T, X₁₁₅ is K or Q, and X₁₁₈ is L, Q or T. For example, the sequence may be a sequence determined according to the rules defined by Chothia.

In some cases, compared with the amino acid sequence set forth in SEQ ID NO: 1, the VH may at least comprise an amino acid substitution at a position selected from the following: amino acid substitutions at X₁, X₁₄, X₁₈, X₄₆, X₄₉, X₅₂, X₅₄, X₅₅, X₅₇, X₆₄, X₇₄, X₇₉, X₈₄, X₈₆, X₈₇, X₉₂, X₁₀₂, X₁₀₃, X₁₀₄, X₁₀₅, X₁₀₆, X₁₀₇, X₁₁₅ and/or X₁₁₈.

For example, the VH may comprise an amino acid sequence set forth in any one of SEQ ID NOs: 1-6.

In the present application, the isolated antigen-binding protein may be VHH. For example, the VHH may comprise an amino acid sequence set forth in any one of SEQ ID NOs: 1-6.

In the present application, a portion of the amino acid sequence of each heavy chain or light chain of the antigen-binding protein is homologous to the corresponding amino acid sequence in an antibody from a specific species, or belongs to a specific class. For example, the variable region and constant portion of a light chain and a heavy chain are both derived from the variable region and constant region of an antibody of one animal species (such as human). In the present application, the homolog may be a protein or a polypeptide that has at least about 85% (e.g., has at least about 85%, about 90%, about 91%, about 92%, about 93%, about 94%, about 95%, about 96%, about 97%, about 98%, about 99% or higher) sequence homology with the amino acid sequence of the protein and/or the polypeptide (e.g., the antibody or fragment thereof that specifically binds to VEGF protein).

In the present application, the homology generally refers to the similarity, resemblance or association between two or more sequences. The alignment for determining the percentage of sequence homology may be achieved in a variety of ways known in the art, for example, by using publicly available computer software such as BLAST, BLAST-2, ALIGN or Megalign (DNASTAR) software. Those skilled in the art may determine appropriate parameters for sequence alignment, including any algorithm required to implement the maximum alignment undergoing comparison, within a full-length sequence range or within a target sequence region. The homology may also be determined by the following methods: FASTA and BLAST. For the description of the FASTA algorithm, a reference may be made to W. R. Pearson and D. J. Lipman, "Improved Tools for Biological Sequence Comparison", Proc. Natl. Acad. Sci.), 85: 2444-2448, 1988; and D. J. Lipman and W. R. Pearson, "Rapid and Sensitive Protein Similarity Searches", Science, 227: 1435-1441, 1989. For the description of the BLAST algorithm, a reference may be made to S. Altschul, W. Gish, W. Miller, E. W. Myers and D. Lipman, "A Basic Local Alignment Search Tool", J. Mol. Biol., 215: 403-410, 1990.

### Nucleic Acid, Vector. Host Cell and Manufacture Method

In another aspect, the present application further provides one or more isolated nucleic acid molecules. The one or more nucleic acids may encode the antigen-binding protein of the present application. For example, each nucleic acid molecule of the one or more nucleic acid molecules may encode the intact antigen-binding protein, and may also encode a part thereof (e.g., one or more of HCDR1-3, LCDR1-3, VL, VH, light chain or heavy chain).

The nucleic acid molecule of the present application may be isolated. For example, it may be produced or synthesized by following methods: (i) amplification *in vitro,* e.g., production by amplification via Polymerase Chain Reaction (PCR), (ii) production by clonal recombination, (iii) purification, e.g., by enzyme cleavage and fraction separation via gel electrophoresis, or (iv) synthesis, e.g., by chemical synthesis. In some embodiments, the isolated nucleic acid is the nucleic acid molecule that is prepared by a DNA recombination technique.

In the present application, the nucleic acids encoding the antibodies or antigen binding fragments thereof may be prepared by various methods known in the art, including but not limited to, using restriction fragment manipulation or using overlapping extension PCR by synthetic oligonucleotides.

In another aspect, the present application provides one or more vectors comprising one or more nucleic acid molecules described in the present application. Each vector may comprise one or more of the nucleic acid molecules. Besides, the vector may also comprise other genes, for example a marker gene that allows the vectors to be selected in an appropriate host cell and under an appropriate condition. Besides, the vector may also comprise an expression control element that allows the coding region to be correctly expressed in an appropriate host. For example, the vector is an expression vector.

In another aspect, the present application provides a host cell, which may comprise one or more nucleic acid molecules of the present application and/or one or more vectors of the present application. In certain embodiments, each type or each of the host cells may comprise one or more nucleic acid molecules or vectors of the present application. In some embodiments, each type or each of the host cells may comprise multiple (e.g., 2 or more) or multiple types of (e.g., 2 or more types of) of the nucleic acid molecules or vectors of the present application.

In another aspect, the present application provides a method for preparing the antibody or antigen-binding fragment thereof. The method may comprise culturing the host cells of the present application under the condition that the antibody or antigen-binding fragment thereof is expressed. For example, it may be achieved by using appropriate medium, appropriate temperature and cultivation time and so on, and these methods are known by those skilled in the art.

### Pharmaceutical Composition, Method, and Use

In another aspect, the present application provides a pharmaceutical composition, which may comprise the antigen-binding protein, the polypeptide, the nucleic acid molecule, the vector, the host cell of the present application, and optionally a pharmaceutically acceptable carrier. The pharmaceutically acceptable adjuvants are non-toxic to recipients at the doses and concentrations employed, and the pharmaceutical composition of the present application may also comprise more than one active compound which are those active compounds having complementary activities without adversely affecting each other. The types and the effective amounts of such medicaments depend on, e.g., the amount and type of the antagonist existing in a formulation, and the clinical parameter of a subject.

The pharmaceutical composition may be used for inhibiting tumor growth. For example, the pharmaceutical composition of the present application may inhibit or delay the development or progression of a disease, reduce the tumor size (even basically eliminate the tumor), and/or alleviate and/or stabilize the disease conditions.

The pharmaceutical composition of the present application may comprise a prophylactically and/or therapeutically effective amount of the antibody, antigen-binding fragment thereof. The prophylactically and/or therapeutically effective amount is the dosage that is required to prevent and/or treat (at least partially treat) a disease or disorder and/or any complications thereof in a subject having or at risk of developing the disease or disorder.

In another aspect, the present application provides the use of the antigen-binding protein and/or the fusion protein in the preparation of a medicament. The medicament is used for treating cancer, inhibiting tumor growth and/or inhibiting tumor cell proliferation. In certain embodiments, the tumor includes a VEGF-overexpressing tumor. In certain embodiments, the tumor includes a VEGFR-overexpressing tumor. In certain embodiments, the tumor includes solid tumors and/or non-solid tumors. In certain embodiments, the tumor includes lung cancer, colorectal cancer, breast cancer, kidney cancer, gastric cancer, liver cancer, blastoma, cervical cancer and/or ovarian cancer.

In another aspect, the present application provides a method for inhibiting the binding of VEGF protein to VEGFR protein, comprising administering the antigen-binding protein and/or the polypeptide of the present application. For example, the method can be an *ex vivo* or *in vitro* method. For example, the method may be a method for non-therapeutic purposes. In some cases, the method may comprise contacting a biological sample with the antigen-binding protein and/or VEGFR of the present application under a condition that allows the binding of the antigen-binding protein and/or VEGFR to VEGF, detecting whether a complex is formed between the antigen-binding protein and VEGF, and detecting whether a complex is formed between VEGF and VEGFR.

In another aspect, the present application provides a method for detecting the presence and/or content of VEGF protein, which comprise administering the isolated antigen-binding protein and/or the polypeptide. For example, the method can be an *ex vivo* or *in vitro* method. For example, the method may be a method for non-therapeutic purpose.

The present application further provides the use of the antigen-binding protein in a method for diagnosing a subject suffering from a tumor or cancer, the method comprising: determining the presence or expression level of VEGF in a sample obtained from a subject by contacting the sample with the antigen-binding protein of the present application and detecting the presence of the bound antibody.

In another aspect, the present application provides an antibody-drug conjugate which may comprise a cytotoxic agent and the antigen-binding fragment of the present application. An antibody-drug conjugate usually refers to an antibody linked to a small molecule cytotoxic drug using a specific linker, the main components of which may comprise an antibody, a linker and a small molecule cytotoxic drug.

In another aspect, the present application provides a kit which may comprise the antigen-binding protein, the chimeric antigen receptor, the genetically modified cell, the antibody-drug conjugate of the present application, and/or the pharmaceutically composition of the present application. It may comprise the antigen-binding protein, the chimeric antigen receptor, the genetically modified cell, and/or the antibody drug conjugate of the present application in a single conventional container, and also may optionally combine with one or more therapeutic agents, optionally they may be formulated together in a pharmaceutical composition.

In another aspect, the present application provides a drug delivery device, which may be used for administration of the antigen-binding protein or pharmaceutical composition thereof described in the present application.

Without intending to be limited by any theory, the following Examples are only used to illustrate the various technical solutions of the invention of the present application, but are not used to limit the scope of the invention of the present application.

### Examples

### Example 1 Preparation and detection of anti-VEGF single domain antibody

The blood samples of three alpacas that had not been immunized with the target antigen were taken, each taking 150ml. Lymphocytes were isolated from the blood samples to extract total RNA, and a phage-displayed nanobody library was constructed with a size of 3×10⁹cfu. 6 ml of transformed antibody library phage were selected to prepare phage for specific panning, and the total amount of phage was 50 times greater than the library capacity.

50ml of Streptavidin Magnetic Beads (Thermo fisher, Cat. No.: 88817) were pre-bound to 1ml of phage and incubated at room temperature for 30min to remove non-specific binding. The background-removed nanobody library phage was selected, to which 10ug Biotinylated Human VEGF165 (ACROBiosystems, Cat. No.: VES-H82Q0), 150ul Streptavidin Magnetic Beads were added, incubated at room temperature for 15min, washed 14 times with PBST (PBS containing 0.05% Tween-20), to wash away unbound phage. The antigen-specifically bound phage was eluted with 450 ul of 100 mM hydrochloric acid, neutralized with addition of 50 ul of 1 M Tris-HCl with pH 11 to infect *E. coli* SS320 in logarithmic growth phase, and phage was produced and purified for the next round of screening. The screening method was the same as the first round, except that the amount of antigen was reduced to 4 ug. The enrichment of enriched phages after two rounds of screening was identified by Enzyme-linked Immunosorbent Assay (ELISA). The results are shown in Table 1 below. The results show that after two rounds of panning, the phages were significantly enriched.

**Table 1 Phage Elisa identification after the second round of enrichment**

| Coated antigen | Sample well | Control well |
|---|---|---|
| OD450 nm absorption value | 1.0713 | 0.1799 |

### Example 2 Single domain antibody screening and in vitro activity identification

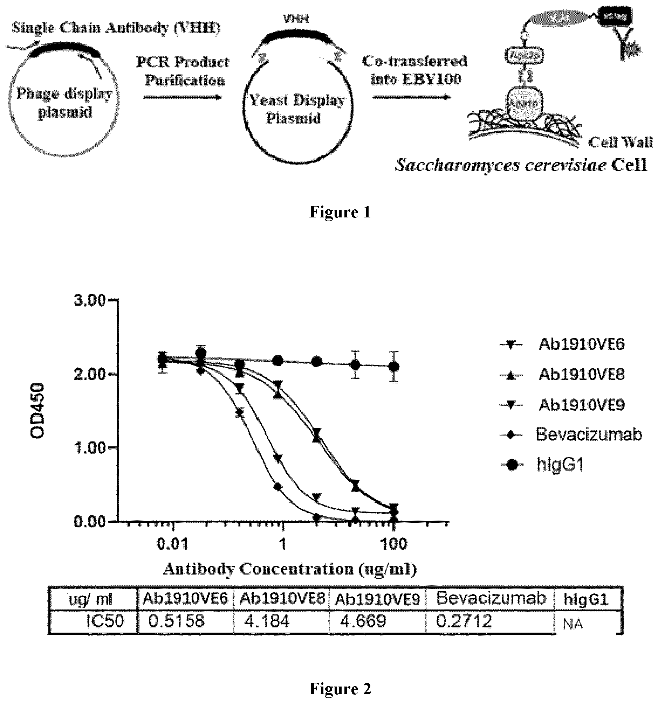

Using the plasmid after two rounds of panning of the single domain antibody library in Example 1 as a template, primers were designed to amplify the nanobody gene (V_{H}H) by Polymerase Chain Reaction (PCR); the PCR-amplified V_{H}H gene fragment was recovered and co-transformed with the yeast display plasmid into *Saccharomyces cerevisiae* strain EBY100 (purchased from ATCC), and the VHH gene was inserted into the yeast display plasmid through homologous recombination *of Saccharomyces cerevisiae,* thereby realizing the display of single domain antibodies on the surface of yeast cell wall. The construction scheme is shown in Figure 1.

The yeast display library was sorted for two rounds using a flow sorter, the yeast obtained by sorting were spread on an auxotrophic plate medium, from which 46 single clones were selected for sequencing, and finally 5 single domain heavy chain antibodies with unique sequences were obtained. The identification protocol is shown in Table 2. The corresponding yeast monoclonal colonies were analyzed by flow staining, and 1×10⁶ cells were selected for staining according to the protocols in Table 2. In Protocol 1, the strength of the cell population that binds to human VEGF165-Bio is reflected by the PE mean fluorescence signal intensity (MFI). Likewise, Protocol 2 and Protocol 3 can be used to assess Mouse VEGF-Bio and non-specific binding level, and in Protocol 4, the competition signal is reflected by APC mean fluorescence signal intensity (MFI). The results are shown in Table 3. As can be seen from Table 3, after exclusion of clones that do not bind to human VEGF or murine VEGF, the monoclone Y20A6 of the anti-human VEGF 165 single domain heavy chain antibody was obtained, and the binding and competition abilities of Y20A6 were both satisfactory.

**Table 2 Identification protocol of flow staining of monoclonal yeast colonies**

| Protocol | Primary antibody | Secondary antibody | Tertiary antibody |
|---|---|---|---|
| 1 | 10 nM Human VEGF-Bio; Mouse Anti V5 (thermo fisher, Cat. No.: R960-25) | Streptavidin-PE; Goat anti-mouse-Alexa Fluor 647 (thermo fisher, Cat. No.: A-21235) | None |
| 2 | 10 nM Mouse VEGF-Bio; Mouse Anti V5 | Streptavidin-PE; Goat anti-mouse-Alexa Fluor 647 | None |
| 3 | 10 nM irrelevant antigen; Mouse Anti V5 | Streptavidin-PE; Goat anti-mouse-Alexa Fluor 647 | None |
| 4 | 10 nM Human VEGF-Bio | 10 nM Bevacizumab | Streptavidin-PE; mouse anti-huamn IgG Fc-APC |

**Table 3 Analysis results of flow staining of yeast monoclonal colonies after the second round of screening of the yeast display library**

| Clone number | Human VEGF binding signal | Murine VEGF binding signal | Irrelevant antigen binding signal | Competition signal |
|---|---|---|---|---|
| Y20A6 | 1477 | 1441 | 21.8 | 25.4 |

### Example 3 Humanization of single domain antibodies

### 3.1 Humanization design and expression of single domain antibodies

The sequence of the monoclonal antibody Y20A6 was subjected to IMGT/Domain Gap Align, and the human germline with the highest homology was found to be IGHV3-23*04. The antibody sequence was numbered according to the Chothia rule, and the sites with different site sequences such as H1, H14, and H18 were mutated as shown in Table 4. The protein numbered Ab1910VE6 was the original antibody before humanization.

**Table 4 Humanization design of single domain antibody sequences**

| Protein number | Chothia | H 1 | H 14 | H 18 | H 44 | H 45 | H 46 | H 49 | H 57 | H 59 | H 60 | H 64 | H 74 | H 79 | H8 2B | H 83 | H 84 | H 89 | H1 05 | H1 08 |
|---|---|---|---|---|---|---|---|---|---|---|---|---|---|---|---|---|---|---|---|---|
| | IGHV3-2 3*04 | E | P | L | G | L | E | S | T | Y | A | K | S | Y | S | H | A | V | Q | L |
| Ab1910 VE6 | Y20A6 | A | A | A | E | R | D | A | R | V | E | R | T | D | N | K | P | I | Q | Q |
| Ab1910 VE8 | Hz20A 6.2 | E | P | L | E | R | E | S | R | V | E | K | S | Y | S | R | A | V | K | T |
| Ab1910 VE9 | Hz20A 6.3 | E | P | L | E | R | E | S | R | V | E | K | S | Y | S | R | A | V | Q | L |

The full-length sequences in Table 4 were fused with IgG1 Fc N297A (purchased from Taizhou Biointron Inc.), codon-optimized, and loaded into the expression vector pcDNA3.4 (Life Technologies) after gene synthesis. After the expression plasmid was amplified and the plasmid was extracted, the plasmid was transferred into ExpiCHO cells (ThermoFisher Scientific, A29133), and the antibody was transiently expressed according to the method of the supplier's ExpiCHO expression system. The purified antibody obtained is shown in Table 5.

**Table 5 Single domain antibody expression and purification data**

| Antibody number | Theoretical isoelectric point | Yield (mg) | Expression amount (mg/L) | Concentration (mg/mL) |
|---|---|---|---|---|
| Ab1910VE6 | 7 | 0.89 | 89 | 4.6 |
| Ab1910VE8 | 7.4 | 0.48 | 48 | 4.8 |
| Ab1910VE9 | 7 | 0.75 | 75 | 3.8 |

### Example 4 Binding activity assay of the antigen-binding protein of the present application to human VEGF165

(1) Octet RED96e (Fortebio) was used to determine the affinity of Ab1910VE6, AB1910VE8 and Ab1910VE9 to human VEGF165 (Aero, Cat. No.: VES-H82Q0). The antigen and antibody were both diluted with 1xPBST (1xPBS: Sango Biotech, B548117-0500; 0.02% Tween 20: sigma-alorich, P1379), the antigen was used at a concentration of 100 nM, and the antibody was used at a concentration of 50 nM.
(2) On-board detection of samples (Octet Data Acquisition 11.1.0.11): First, the samples were added to a 96-well plate (Greiner bio-one, 655209), and the system was 200 µL/well. Then the software parameters were set, the plate temperature was set to be 30°C, and the frequency of collecting the standard kinetic signal was 5.0 Hz. Next, the AHC sensor (Fortébio, Cat. No.: 18-0015) was pre-wetted with 1×PBST for 10 minutes, and then detected on the machine. Each cycle consists of the following steps: 1) immersion in the buffer for 60 seconds; 2) detection of whether there is a non-specific binding bewteen antigen and sensor; 3) regeneration of 10 mM glycine solution with pH 1.7; 4) immersion in the buffer for 60 seconds; 5) immobilization of the antibody on the sensor for a time of 20 seconds; 6) immersion of the sensor in the buffer for 180 seconds; 7) binding of the antigen to the antibody for a time of 180 seconds; 8) dissociation of the antigen and the antibody for 10 minutes; 9) sensor regeneration.

### (3) Data analysis

Fortebio's Data Analysis 11.0 software was used to measure the binding rate (Ka) and dissociation rate (Kd) of the antigen-antibody in a 1:1 binding mode, and then the equilibrium dissociation constant (KD) of the antibody was calculated. The results are shown in Table 6. Ab1910VE6, AB1910VE8 and Ab1910VE9 all have a strong affinity to VEGF. This shows that the antigen-binding protein of the present application has a high binding affinity to VEGF.

**Table 6 Affinity of single domain antibodies to human VEGF 165**

| Candidate antibody | Response value | *K_{D}* (M) | kon(1/Ms) | kdis(1/s) |
|---|---|---|---|---|
| AB1910VE6 | 0.4416 | 6.304E-09 | 1.41E+06 | 8.88E-03 |
| AB1910VE8 | 0.4095 | 1.052E-08 | 1.75E+06 | 1.84E-02 |
| AB1910VE9 | 0.4062 | 9.913E-09 | 1.82E+06 | 1.81E-02 |
| Bevacizumab | 0.3414 | <1.0E-12 | 6.75E+05 | <1.0E-07 |

### Example 5 Activity assay of the antigen-binding protein of the present application in blocking the binding of human VEGF 165 to human VEGFR2

A competitive ELISA method was used to determine the activity of the single domain antibody in blocking the binding of VEGF165 to human VEGFR2. The specific steps were carried out as follows: Human VEGF R2 (Aero, Cat. No.: KDR-H5227) was diluted with PBS (Gibco, Cat. No.: 10010-023) to 1 µg/ mL, added to a 96-well plate, 100 µL per well, affixed with a sealing film, and incubated overnight at 4°C. The next day, the plate was washed 3 times with washing solution PBST (0.05% TWEEN-20: sigma-alorich, P1379), and then the blocking solution PBST+2% BSA (BSA: VWR, 0332-1KG) was prepared using the washing solution, with 300 µL of blocking solution added to each well, blocked at 37°C for 1 hour. Anti-VEGF and Biotin-hVEGF (Aero, Cat. No.: VES-H82Q0) were prepared with the blocking solution, the initial formulated concentration of Anti-VEGF was 200 µg/mL, followed by 5-fold serial dilution (7 concentration points + 1 zero concentration point), the formulated concentration of Biotin-hVEGF was 90 ng/mL. Afterwards, the sealed ELISA plate was taken out, the plate was washed three times with the washing solution, 50 µL of diluted Anti-VEGF were selected and added to the 96-well plate, and then 50 µL of Biotin-hVEGF were selected and added to the 96-well plate, and co-incubated at 37°C for 1 hour. The plate was washed three times with the washing solution, the secondary antibody SA-HRP (sigma, S2438) was diluted 1:5000 with the blocking solution, added to the ELISA plate according to 100 µL/well, and incubated at 37°C for 1 hour. The plate was washed 3 times with the washing solution, 100 µL of TMB chromogenic solution (Biopanda, Cat. No.: TMB-S-003) were added to each well, and developed color at room temperature in the dark, and then 50 µL of stop solution (Solarbio, Cat. No.: C1058) were added to each well to stop the reaction, and the absorbance was measured at a wavelength of 450 nm. The results showed (Figure 2) that Ab1910VE8, Ab1910VE9 and Ab1910VE6 all had strong blocking abilities. This shows that the antigen-binding protein of the present application has a good effect in blocking the binding of VEGF to VEGFR.

### Example 6 Affinity maturation and in vitro identification of the antigen-binding protein of the present application

### 6.1 Construction of affinity-matured library of single domain antibody

Ab1910VE9 sequence was selected for affinity maturation, and the CDR regions of Ab1910VE9 were defined by Chothia. For variable regions CDR1, CDR2, and CDR3, NNK mutation primers were designed for Polymerase Chain Reaction (PCR) to amplify gene fragments of each CDR mutation library. According to the method described in the literature, each CDR mutation library gene fragment and yeast display plasmid were respectively transformed into *Saccharomyces cerevisiae* strain EBY100 (purchased from ATCC), so that each CDR mutation library was displayed on the surface of yeast. At the same time, the parental sequence of Ab1910VE9 was displayed on the surface of yeast and used as a control.

After the library was cultured and induced, Biotin-Human VEGF 165 was used for three rounds of sorting, starting at an initial concentration of 3 nM and subjected to a 10-fold serial dilution. The cell population with high display level and strong antigen-binding ability was collected; after sorting, the cells were spread on SD-Trp solid medium and subjected to static culture at 30°C for 3 days.

### 6.2 Monoclonal identification

Single clones were picked and sent for sequencing, and the obtained single clones were identified by flow staining, incubated with 1nM Biotin-Human VEGF165 for staining, and the ratio of the displayed mean fluorescence signal intensity to the antigen-binding mean fluorescence signal intensity in different clones was compared, reflecting the capacity of a single molecule to bind to an antigen. According to the binding force value, a total of 3 molecules with different mutation sequences in each CDR region were selected for subsequent *in vitro* evaluation, as shown in Table 7 for details.

**Table 7 Monoclonal identification results and antibody expression numbers**

| Antibody number | Clone number | 1 nM Human VEGF165-Biotin | | | Annotation |
|---|---|---|---|---|---|
| | | Displaying MFI | Binding MFI | Binding force | |
| Ab1910VE18 | Ab1910VE9 | 3583 | 42.1 | 11.7 | Parent sequence |
| Ab1910VE21 | YC41D1 | 3431 | 337 | 98.2 | Affinity-matured mutant sequence |
| Ab1910VE23 | YC43H2 | 2770 | 358 | 129.2 | |
| Ab1910VE24 | YC43B1 | 4395 | 503 | 114.4 | |

### 6.3 Expression of affinity-matured single domain antibody and SEC-HPLC purity analysis

Each single domain antibody in Table 7 was expressed and purified. The expressed antibody was subjected to SEC-HPLC purity analysis, with the method being as follows:
(1) The sample was diluted to 1 mg/mL, mixed well, centrifuged at 12,000 rpm for 5 min, the supernatant was selected and transferred to a sample bottle and placed into the HPLC sample tray. The chromatographic conditions were set as follows:

| Chromatographic condition | Parameter |
|---|---|
| Chromatographic column | TSK G3000SWxl |
| Detection wavelength | 280nm |
| Column temperature | 25°C |
| Sample chamber temperature | 5°C |
| Flow rate | 0.5ml/min |

(2) The chromatographic column was equilibrated with mobile phase (200 mM phosphate buffer, pH 6.8), injected and analyzed, and data analysis was performed with chromatographic software, and the peak area percentage of each peak was calculated by the peak area normalization method.

The specific results are shown in Table 8.

**Table 8 Expression and purification data of affinity-matured single domain antibodies**

| Antibody number | Theoretical isoelectric point | Yield (mg) | Expression (mg/L) | Concentration (mg/ml) | Monomer rate (%) |
|---|---|---|---|---|---|
| Ab1910VE18 | 7.5 | 3.26 | 130 | 2.17 | 96.4 |
| Ab1910VE21 | 7.6 | 3.54 | 142 | 2.36 | 96.9 |
| Ab1910VE23 | 7.5 | 2.54 | 101 | 1.69 | 96.2 |
| Ab1910VE24 | 7.3 | 2.79 | 112 | 1.86 | 96.5 |
| | | | | | |

### Example 7 Determination of kinetic parameters of the antigen-binding protein of the present application

Referring to the method in Example 4, the affinity of the antibody to human VEGF 165 was determined before and after affinity maturation.

**Table 9 Affinity of affinity-matured antibody to human VEGF165**

| Antibody of the present application | Response value | KD (M) | kon(1/Ms) | kdis(1/s) |
|---|---|---|---|---|
| Ab1910VE18 | 0.1421 | 2.227E-09 | 1.81E+06 | 4.03E-03 |
| Ab1910VE21 | 0.1536 | 4.126E-10 | 1.24E+06 | 5.10E-04 |
| Ab1910VE23 | 0.1164 | 7.276E-10 | 1.43E+06 | 1.04E-03 |
| Ab1910VE24 | 0.1532 | 6.257E-10 | 1.37E+06 | 8.57E-04 |
| Bevacizumab | 0.2211 | <1.0E-12 | 3.50E+05 | <1.0E-07 |

The results are shown in Table 9. Ab1910VE18 is a molecule before affinity maturation, and after maturation, the affinity of the antibody molecule is increased by about 2-5 times. This shows that the antigen-binding protein of the present application has a high binding affinity to VEGF.

### Example 8 Experiment on the function of the antigen-binding protein of the present application to block the binding of human VEGF165 to human VEGFR2

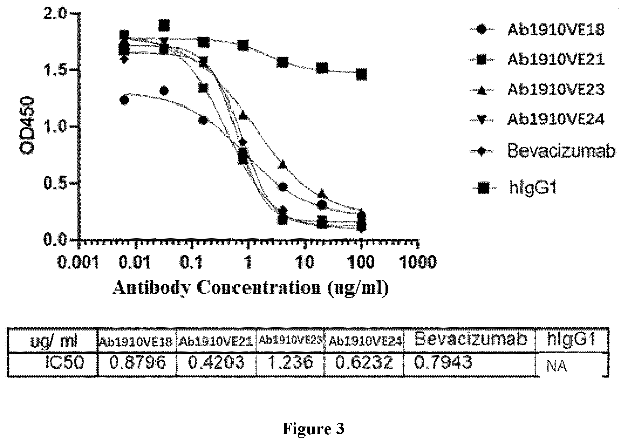

Referring to the method of Example 5, the experiment for determining the function of affinity-matured VEGF antibody to block the binding of human VEGF165 to human VEGFR2 was determined, wherein the formulated concentration of Biotin-hVEGF165 was 700ng/mL. The experimental results are shown in Figure 3. The blocking effects of Ab1910VE18, Ab1910VE21, and Ab1910VE24 antibodies are comparable to those of Bevacizumab. This shows that the antigen-binding protein of the present application has a good effect in blocking the binding of VEGF to VEGFR.

The foregoing detailed description is provided by way of explanation and examples, and is not intended to limit the scope of the appended claims. Various changes of the embodiments listed in the present application are obvious to those of ordinary skills in the art, and are reserved within the scope of the appended claims and the equivalent solutions thereof.

## Claims

1. An isolated antigen-binding protein having one or more of the following properties:
(1) capable of binding VEGF protein with *a K_{D}* value of 1×10⁻⁷ M or lower; and
(2) capable of blocking the binding of VEGF protein to VEGFR protein.

2. The isolated antigen-binding protein according to claim 1, wherein the VEGF protein includes VEGF165.

3. The isolated antigen-binding protein according to any one of claims 1-2, wherein the VEGFR protein includes VEGFR2.

4. The isolated antigen-binding protein according to any one of claims 1-3, which includes an antibody or an antigen binding fragment thereof.

5. The isolated antigen-binding protein according to claim 4, wherein the antigen binding fragment includes Fab, Fab', F(ab)₂, Fv fragment, F(ab')₂, scFv, di-scFv, VHH and/or dAb.

6. The isolated antigen-binding protein according to claim 4 or 5, wherein the antigen binding fragment is VHH.

7. The isolated antigen-binding protein according to any one of claims 4-6, wherein the antibody is selected from the group consisting of: a monoclonal antibody, a chimeric antibody, a humanized antibody and a fully human antibody.

8. The isolated antigen-binding protein according to any one of claims 1-7, which is capable of competing with a reference antibody for binding to the VEGF protein, wherein the reference antibody comprises a heavy chain variable region VH, the VH of the reference antibody comprises HCDR1, HCDR2 and HCDR3, and the HCDR1 of the reference antibody comprises an amino acid sequence set forth in SEQ ID NO: 9, the HCDR2 of the reference antibody comprises an amino acid sequence set forth in SEQ ID NO: 23, and, the HCDR3 of the reference antibody comprises an amino acid sequence set forth in SEQ ID NO: 24.

9. The isolated antigen-binding protein according to claim 8, wherein the HCDR1 of the reference antibody comprises an amino acid sequence set forth in SEQ ID NO: 9.

10. The isolated antigen-binding protein according to any one of claims 8-9, wherein the HCDR2 of the reference antibody comprises an amino acid sequence set forth in any one of SEQ ID NOs: 12-14.

11. The isolated antigen-binding protein according to any one of claims 8-10, wherein the HCDR3 of the reference antibody comprises an amino acid sequence set forth in any one of SEQ ID NOs: 17-19.

12. The isolated antigen-binding protein according to any one of claims 1-11, which comprises at least one CDR in a VH, the VH comprising an amino acid sequence set forth in SEQ ID NO: 29.

13. The isolated antigen-binding protein according to any one of claims 1-12, which comprises at least one CDR in a VH, the VH comprising an amino acid sequence set forth in any one of SEQ ID NOs: 1-6.

14. The isolated antigen-binding protein according to any one of claims 1-13, which comprises HCDR3, and the HCDR3 comprises an amino acid sequence set forth in SEQ ID NO: 24.

15. The isolated antigen-binding protein according to claim 14, wherein the HCDR3 comprises an amino acid sequence set forth in any one of SEQ ID NOs: 17-19.

16. The isolated antigen-binding protein according to any one of claims 1-15, which comprises HCDR2, and the HCDR2 comprises an amino acid sequence set forth in SEQ ID NO: 23.

17. The isolated antigen-binding protein according to claim 16, wherein the HCDR2 comprises an amino acid sequence set forth in any one of SEQ ID NOs: 12-14.

18. The isolated antigen-binding protein according to any one of claims 1-17, which comprises HCDR1, and the HCDR1 comprises an amino acid sequence set forth in SEQ ID NO: 9.

19. The isolated antigen-binding protein according to any one of claims 1-18, which comprises HCDR1, HCDR2 and HCDR3, wherein the HCDR1 comprises an amino acid sequence set forth in SEQ ID NO: 9, the HCDR2 comprises an amino acid sequence set forth in SEQ ID NO: 23, and the HCDR3 comprises an amino acid sequence set forth in SEQ ID NO: 24.

20. The isolated antigen-binding protein according to any one of claims 1-19, which comprises HCDR1, HCDR2 and HCDR3, wherein the HCDR1 comprises an amino acid sequence set forth in SEQ ID NO: 9, the HCDR2 comprises an amino acid sequence set forth in any one of SEQ ID NOs: 12-14, and the HCDR3 comprises an amino acid sequence set forth in any one of SEQ ID NOs: 17-19.

21. The isolated antigen-binding protein according to any one of claims 1-20, which comprises HCDR1, HCDR2 and HCDR3, and the HCDR1, HCDR2 and HCDR3 are selected from any one of the following groups of amino acid sequences:
(1) HCDR1: SEQ ID NO: 9, HCDR2: SEQ ID NO: 12 and HCDR3: SEQ ID NO: 17;
(2) HCDR1: SEQ ID NO: 9, HCDR2: SEQ ID NO: 13 and HCDR3: SEQ ID NO: 17;
(3) HCDR1: SEQ ID NO: 9, HCDR2: SEQ ID NO: 14 and HCDR3: SEQ ID NO: 17;
(4) HCDR1: SEQ ID NO: 9, HCDR2: SEQ ID NO: 13 and HCDR3: SEQ ID NO: 18; and
(5) HCDR1: SEQ ID NO: 9, HCDR2: SEQ ID NO: 13 and HCDR3: SEQ ID NO: 19.

22. The isolated antigen-binding protein according to any one of claims 1-21, which comprises a heavy chain variable region VH, wherein the VH comprises a framework region H-FR1, the C-terminus of the H-FR1 is directly or indirectly linked to the N-terminus of the HCDR1, and the H-FR1 comprises an amino acid sequence set forth in SEQ ID NO: 25.

23. The isolated antigen-binding protein according to claim 22, wherein the H-FR1 comprises an amino acid sequence set forth in any one of SEQ ID NOs: 7-8.

24. The isolated antigen-binding protein according to any one of claims 22-23, wherein the VH comprises a framework region H-FR2, the H-FR2 is located between the HCDR1 and the HCDR2, and the H-FR2 comprises an amino acid sequence set forth in SEQ ID NO: 26.

25. The isolated antigen-binding protein according to claim 24, wherein the H-FR2 comprises an amino acid sequence set forth in any one of SEQ ID NOs: 10-11.

26. The isolated antigen-binding protein according to any one of claims 22-25, wherein the VH comprises a framework region H-FR3, the H-FR3 is located between the HCDR2 and the HCDR3, and the H-FR3 comprises an amino acid sequence set forth in SEQ ID NO: 27.

27. The isolated antigen-binding protein according to claim 26, wherein the H-FR3 comprises an amino acid sequence set forth in any one of SEQ ID NO: 15-16.

28. The isolated antigen-binding protein according to any one of claims 22-27, wherein the N-terminus of the H-FR4 is linked to the C-terminus of the HCDR3, and the H-FR4 comprises an amino acid sequence set forth in SEQ ID NO: 28.

29. The isolated antigen-binding protein according to claim 28, wherein the H-FR4 comprises an amino acid sequence set forth in any one of SEQ ID NOs: 20-22.

30. The isolated antigen-binding protein according to any one of claims 1-29, which comprises H-FR1, H-FR2, H-FR3 and H-FR4, wherein the H-FR1 comprises an amino acid sequence set forth in SEQ ID NO: 25, the H-FR2 comprises an amino acid sequence set forth in SEQ ID NO: 26, the H-FR3 comprises an amino acid sequence set forth in SEQ ID NO: 27 and the H-FR4 comprises an amino acid sequence set forth in SEQ ID NO: 28.

31. The isolated antigen-binding protein according to any one of claims 1-30, which comprises H-FR1, H-FR2, H-FR3 and H-FR4, wherein the H-FR1 comprises an amino acid sequence set forth in SEQ ID NO: 7 or SEQ ID NO: 8, the H-FR2 comprises an amino acid sequence set forth in SEQ ID NO: 10 or SEQ ID NO: 11, the H-FR3 comprises an amino acid sequence set forth in SEQ ID NO: 15 or SEQ ID NO: 16, and the H-FR4 comprises an amino acid sequence set forth in any one of SEQ ID NOs: 20-22.

32. The isolated antigen-binding protein according to any one of claims 1-31, which comprises H-FR1, H-FR2, H-FR3 and H-FR4, and the H-FR1, H-FR2, H-FR3 and H-FR4 are selected from any one of the following groups of amino acid sequences:
(1) F-FR1: SEQ ID NO: 7, H-FR2: SEQ ID NO: 10, H-FR3: SEQ ID NO: 15 and H-FR4: SEQ ID NO: 20;
(2) H-FR1: SEQ ID NO: 8, H-FR2: SEQ ID NO: 11, H-FR3: SEQ ID NO: 16 and H-FR4: SEQ ID NO: 21; and
(3) H-FR1: SEQ ID NO: 8, H-FR2: SEQ ID NO: 11, H-FR3: SEQ ID NO: 16 and H-FR4: SEQ ID NO: 22.

33. The isolated antigen-binding protein according to any one of claims 1-32, which comprises a heavy chain variable region VH, and the VH comprises an amino acid sequence set forth in SEQ ID NO: 29.

34. The isolated antigen-binding protein according to claim 33, wherein the VH comprises an amino acid sequence set forth in any one of SEQ ID NOs: 1-6.

35. One or more isolated nucleic acid molecules encoding the isolated antigen-binding protein of any one of claims 1-34.

36. A vector comprising the nucleic acid molecule of claim 35.

37. A cell comprising the nucleic acid molecule of claim 35 or the vector of claim 36.

38. A method for preparing the isolated antigen-binding protein of any one of claims 1-34, the method comprising culturing the cell of claim 37 under such a condition that the isolated antigen-binding protein of any one of claims 1-34 is expressed.

39. A pharmaceutical composition comprising the isolated antigen-binding protein of any one of claims 1-34, the nucleic acid molecule of claim 35, the vector of claim 36 and/or the cell of claim 37, and optionally a pharmaceutically acceptable carrier.

40. A polypeptide comprising the isolated antigen-binding protein of any one of claims 1-34.

41. An immunoconjugate comprising the isolated antigen-binding protein of any one of claims 1-34 or the polypeptide of claim 40.

42. Use of the isolated antigen-binding protein of any one of claims 1-34, the nucleic acid molecule of claim 35, the vector of claim 36, the cell of claim 37, the pharmaceutical composition of claim 39, the polypeptide of claim 40 and/or the immunoconjugate of claim 41 in the preparation of a medicament for preventing, alleviating and/or treating tumors.

43. The use according to claim 42, wherein the tumor includes a VEGF-overexpressing tumor.

44. The use according to claim 42 or 43, wherein the tumor includes a solid tumor and/or a non-solid tumor.

45. The use according to any one of claims 42-44, wherein the tumor includes lung cancer, colorectal cancer, breast cancer, kidney cancer, gastric cancer, liver cancer, blastoma, cervical cancer and/or ovarian cancer.

46. A method for preventing, alleviating or treating tumors, the method comprising administering the isolated antigen-binding protein of any one of claims 1-34, the nucleic acid molecule of claim 35, the vector of claim 36, the cell of claim 37, the pharmaceutical composition of claim 39, the polypeptide of claim 40 and/or the immunoconjugate of claim 41 to a subject in need thereof.

47. The method according to claim 46, wherein the tumor includes a VEGF-overexpressing tumor.

48. The method of claim 46 or 47, wherein the tumor includes a solid tumor and/or a non-solid tumor.

49. The method according to any one of claims 46-48, wherein the tumor includes lung cancer, colorectal cancer, breast cancer, kidney cancer, gastric cancer, liver cancer, blastoma, cervical cancer and/or ovarian cancer.

50. The isolated antigen-binding protein of any one of claims 1-34, the nucleic acid molecule of claim 35, the vector of claim 36, the cell of claim 37, the pharmaceutical composition of claim 39, the polypeptide of claim 40 and/or the immunoconjugate of claim 41 for use in preventing, alleviating or treating tumors.

51. The isolated antigen-binding protein, nucleic acid molecule, vector, cell, pharmaceutical composition, polypeptide and/or immunoconjugate according to claim 50, wherein the tumor includes a VEGF-overexpressing tumor.

52. The isolated antigen-binding protein, nucleic acid molecule, vector, cell, pharmaceutical composition, polypeptide and/or immunoconjugate according to claim 50 or 51, wherein the tumor includes a solid tumor and/or a non-solid tumor.

53. The isolated antigen-binding protein, nucleic acid molecule, vector, cell, pharmaceutical composition, polypeptide and/or immunoconjugate according to any one of claims 50-52, wherein the tumor includes lung cancer, colorectal cancer, breast cancer, kidney cancer, gastric cancer, liver cancer, blastoma, cervical cancer and/or ovarian cancer.

54. A method for inhibiting the binding of VEGF protein to VEGFR protein, comprising administering the isolated antigen-binding protein of any one of claims 1-34, the nucleic acid molecule of claim 35, the vector of claim 36, the cell of claim 37, the pharmaceutical composition of claim 39, the polypeptide of claim 40 and/or the immunoconjugate of claim 41.
